# EUROPEAN PATENT SPECIFICATION

(11) **EP 4 070 713 B1**
(45) Date of publication and mention of the grant of the patent: **15.04.2026**
(21) Application number: 20897164.8
(22) Date of filing: 22.10.2020
(51) Int. Cl.: A61B 1/00, A61B 1/045, A61B 1/05, A61B 1/06, A61B 1/07

(54) **ENDOSCOPE SYSTEM AND CONTROL PROGRAM**
ENDOSKOPSYSTEM UND STEUERPROGRAMM
SYSTÈME D'ENDOSCOPE ET PROGRAMME DE COMMANDE

(30) Priority: 02.12.2019 JP 2019218060
(43) Date of publication of application: 12.10.2022
(73) Proprietor: FUJIFILM Corporation, Tokyo 106-8620 (JP)
(72) Inventor: SHIMOMURA Koji, Ashigarakami-gun, Kanagawa 258-8538 (JP)
(74) Representative: Dehns Germany Partnerschaft mbB
(86) International application number: PCT/JP2020/039696
(87) International publication number: WO 2021/111756

(56) References cited:
- WO-A1-2010/122823
- WO-A1-2017/068650
- WO-A1-2018/069992
- WO-A1-2018/116572
- WO-A1-2018/135041
- WO-A1-2018/135041
- WO-A1-2018/180631
- WO-A1-2019/078237
- JP-A- 2001 046 318
- JP-A- 2008 264 313
- JP-A- 2012 509 715
- JP-A- 2018 139 847
- US-A1- 2008 004 529
- US-A1- 2019 290 108

## Description

### BACKGROUND OF THE INVENTION

### 1. Field of the Invention

The present invention relates to an endoscope system, a control program, and a display method.

### 2. Description of the Related Art

In the related art, an endoscope system comprising an imaging element at a distal end part of an endoscope and capable of displaying a live image obtained by the imaging element is known. In addition, JP2005-77832A discloses an industrial endoscope system that automatically determines that a distal end part of an endoscope reaches a target position based on a live image and switches a display screen.

JP2014-83289A discloses an insertion support device that determines whether or not a distal end of an insertion part reaches a predetermined position based on a captured image and switches a state of display output. JP2009-226170A discloses a medical image processing device that changes a video displayed on a monitor in a mode in accordance with a usage state of an external medical device. Endoscope systems are also disclosed in US 2019/290108 A1, JP 2018 139847 A and WO 2018/135041 A1. US 2019/290108 A1 describes, in particular, a standard endoscope system comprising a determination unit configured to determine a stage of an examination by an endoscope and a display unit configured to switch a display image in accordance with a determination result by the determination unit.

### SUMMARY OF THE INVENTION

Generally, in an examination of an inside of a large intestine using an endoscope, after inserting an insertion part of the endoscope into a subject, the inside of the subject is observed while pulling out the insertion part of the endoscope from the subject. In such an examination, useful information for an operator, such as a doctor, of the endoscope varies between a stage of inserting the insertion part of the endoscope into the subject and a stage of performing the observation while pulling out the insertion part of the endoscope from the subject.

For example, in the stage of inserting the insertion part of the endoscope into the subject, it is necessary not to apply an excessive load to the subject, so that the difficulty of operating the endoscope is high, and information for supporting smooth insertion of the insertion part of the endoscope into the subject is useful.

On the other hand, in the stage of performing the observation while pulling out the insertion part of the endoscope from the subject, the difficulty of operating the endoscope is not as high as in a case of insertion, but information for supporting the observation is useful such that a singular portion is not overlooked in the observation based on the live image or the like.

However, the related art described above cannot provide useful information in accordance with a stage of the examination to the operator of the endoscope.

The present invention has been made in view of the circumstances described above, and is to provide an endoscope system, a control program, and a display method that can provide useful information in accordance with a stage of the examination to an operator of an endoscope.

An aspect of the present invention relates to an endoscope system as defined by claim 1.

Another aspect of the present invention relates to a control program causing a computer to function as the determination unit and the display unit of the endoscope system described above.

According to the present invention, it is possible to provide the endoscope system and the control program that can provide useful information in accordance with the stage of the examination to the operator of the endoscope.

### BRIEF DESCRIPTION OF THE DRAWINGS

Fig. 1 is a view showing an example of an endoscope apparatus 100 which is one embodiment of the present invention.
Fig. 2 is a schematic view showing an internal configuration of the endoscope apparatus 100 shown in Fig. 1.
Fig. 3 is a diagram showing an example of functional blocks of a system control unit 44 of a control device 4 shown in Fig. 2.
Fig. 4 is a diagram showing an example of a screen displayed on the display device 7 in an insertion stage of an endoscope 1 in a first embodiment.
Fig. 5 is a diagram showing an example of a screen displayed on the display device 7 in a pulling-out stage of the endoscope 1 in the first embodiment.
Fig. 6 is a flowchart showing an example of display control processing by the control device 4 in the first embodiment.
Fig. 7 is a diagram showing an example of a screen displayed on the display device 7 in an esophagus insertion stage of the endoscope 1 in a second embodiment.
Fig. 8 is a diagram showing an example of a screen displayed on the display device 7 in a stomach insertion stage of the endoscope 1 in the second embodiment.
Fig. 9 is a flowchart showing an example of the display control processing of the control device 4 in the second embodiment.
Fig. 10 is a diagram showing an example of a light source device 5 capable of switching illumination light having different emission spectra and performing irradiation.
Fig. 11 is a diagram showing an example of a spectrum of light generated by the light source device 5 shown in Fig. 10.

### DESCRIPTION OF THE PREFERRED EMBODIMENTS

In the following, embodiments of the present invention will be described with reference to the drawings.

### (First Embodiment)

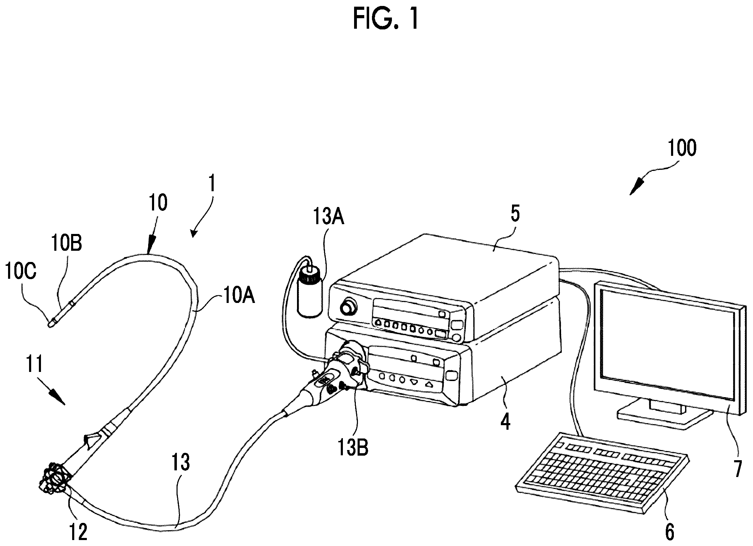

Fig. 1 is a view showing an example of an endoscope apparatus 100 which is one embodiment of the present invention.

The endoscope apparatus 100 is an example of an endoscope system according to the embodiment of the present invention. As shown in Fig. 1, the endoscope apparatus 100 comprises an endoscope 1, and a control device 4 and a light source device 5 to which the endoscope 1 is connected.

A display device 7 that displays a captured image or the like obtained by imaging an inside of a subject by the endoscope 1 and an input unit 6, which is an interface for inputting various pieces of information to the control device 4 are connected to the control device 4. The control device 4 controls the endoscope 1, the light source device 5, and the display device 7.

The display device 7 has a display surface on which display pixels are two-dimensionally arranged, and pixel data constituting image data is drawn on each display pixel on the display surface, thereby performing the display of an image based on the image data. The display device 7 constitutes a display unit that switches the display image in accordance with the command from the control device 4.

The endoscope 1 includes an insertion part 10 which is a tubular member extending in one direction and is inserted into the subject, an operating part 11 which is provided in a base end part of the insertion part 10 and includes an operation member for performing an observation mode switching operation, an imaging recording operation, a forcep operation, an air supply/water supply operation, and a suction operation, an angle knob 12 provided adjacent to the operating part 11, and a universal cord 13 including connector portions 13A and 13B that detachably connect the endoscope 1 to the light source device 5 and the control device 4, respectively.

It should be noted that, although not shown in Fig. 1, various channels, such as a forcep hole for inserting forceps for sampling a living body tissue, such as cells or polyps, an air supply/water supply channel, and a suction channel, are provided inside the operating part 11 and the insertion part 10.

The insertion part 10 is composed of a flexible part 10A having flexibility, a bendable part 10B provided at a distal end of the flexible part 10A, and a hard distal end part 10C provided at a distal end of the bendable part 10B.

The bendable part 10B is configured to be bendable by a rotational movement operation of the angle knob 12. Depending on the site of the subject in which the endoscope 1 is used, the bendable part 10B can be bent in any direction and at any angle, and the distal end part 10C can be directed in a desired direction.

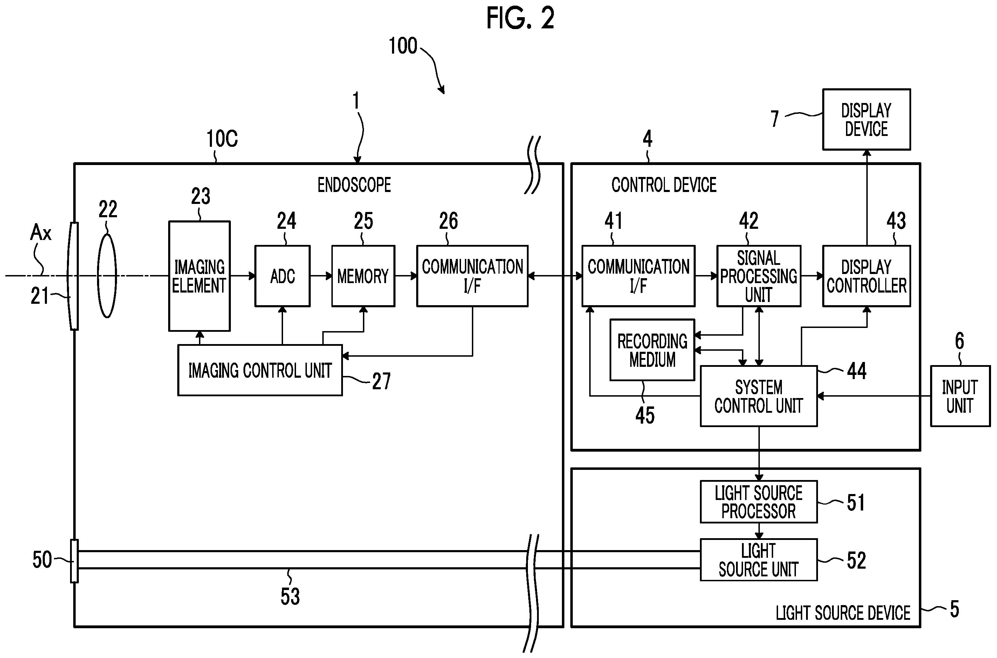

Fig. 2 is a schematic view showing an internal configuration of the endoscope apparatus 100 shown in Fig. 1.

The light source device 5 comprises a light source processor 51 and a light source unit 52. The light source unit 52 emits illumination light to be emitted to the subject. The illumination light emitted from the light source unit 52 enters a light guide 53 built in the universal cord 13, and is emitted to the subject through an illumination lens 50 provided at the distal end part 10C of the insertion part 10.

A white light source that emits white light, a plurality of light sources including the white light source and a light source (for example, blue light source that emits blue light) that emits other color light, or the like is used as the light source unit 52. A plurality of illumination lenses 50 may be provided in accordance with the type of light emitted from the light source unit 52 on a distal end surface of the distal end part 10C.

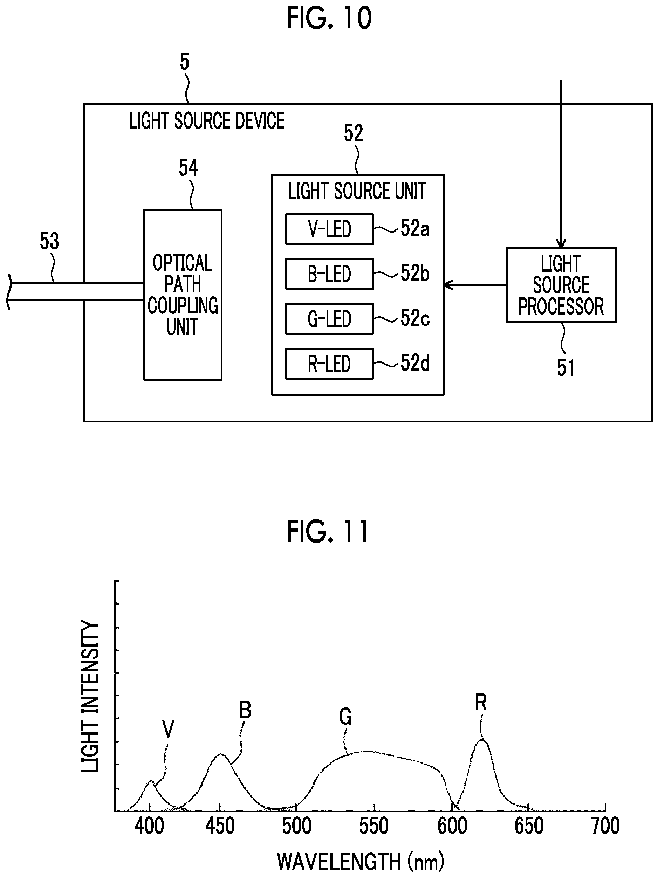

In addition, the light source unit 52 may be capable of switching the illumination light having different emission spectra and performing irradiation. A specific configuration of the light source unit 52 capable of switching the illumination light having different emission spectra and performing the irradiation will be described with reference to Figs. 10 and 11.

The light source processor 51 is connected to a system control unit 44 of the control device 4. The light source processor 51 controls the light source unit 52 based on the command from the system control unit 44.

In the distal end part 10C of the endoscope 1, an imaging optical system including an objective lens 21 and a lens group 22, an imaging element 23 that images the subject through the imaging optical system, an analog/digital conversion circuit (ADC) 24, a memory 25, such as a random access memory (RAM), a communication interface (I/F) 26, an imaging control unit 27, and the light guide 53 for guiding the illumination light emitted from the light source unit 52 to the illumination lens 50 are provided.

The light guide 53 extends from the distal end part 10C to the connector portion 13A of the universal cord 13. The illumination light emitted from the light source unit 52 of the light source device 5 can enter the light guide 53 in a state in which the connector portion 13A of the universal cord 13 is connected to the light source device 5.

A charge coupled device (CCD) image sensor or a complementary metal oxide semiconductor (CMOS) image sensor is used as the imaging element 23.

The imaging element 23 has a light-receiving surface on which a plurality of pixels are two-dimensionally arranged, converts an optical image formed on the light-receiving surface by the imaging optical system described above into an electrical signal (imaging signal) in each pixel, and outputs the converted electrical signal to the ADC 24. For example, an imaging element on which a color filter, such as an elementary color or a complementary color, is mounted, is used as the imaging element 23. A set of the imaging signals output from the pixels of the light-receiving surface of the imaging element 23 is referred to as a captured image signal.

It should be noted that, in a case in which a light source unit in which the spectrum of the white light emitted from the white light source is divided in a time-division manner by a plurality of color filters to generate the illumination light is used as the light source unit 52, an imaging element on which no color filter is mounted may be used as the imaging element 23.

The imaging element 23 may be disposed at the distal end part 10C in a state in which the light-receiving surface is perpendicular to an optical axis Ax of the objective lens 21, or may be disposed at the distal end part 10C in a state in which the light-receiving surface is parallel to the optical axis Ax of the objective lens 21.

The imaging optical system provided in the endoscope 1 is composed of optical members (including the lens group 22 described above), such as a lens and a prism, which are present on an optical path of the light from the subject between the imaging element 23 and the objective lens 21, and the objective lens 21. There is also a case in which the imaging optical system is composed of only the objective lens 21.

The ADC 24 converts the imaging signal output from the imaging element 23 into a digital signal having a predetermined number of bits.

The memory 25 transitorily records the imaging signal digitally converted by the ADC 24.

The communication I/F 26 is connected to a communication interface (I/F) 41 of the control device 4. The communication I/F 26 transmits the imaging signal recorded in the memory 25 to the control device 4 through a signal line in the universal cord 13.

The imaging control unit 27 is connected to the system control unit 44 of the control device 4 via the communication I/F 26. The imaging control unit 27 controls the imaging element 23, the ADC 24, and the memory 25 based on the command from the system control unit 44 received by the communication I/F 26.

The control device 4 comprises the communication I/F 41, which is connected to the communication I/F 26 of the endoscope 1 by the universal cord 13, a signal processing unit 42, a display controller 43, the system control unit 44, and a recording medium 45.

The communication I/F 41 receives the imaging signal transmitted from the communication I/F 26 of the endoscope 1 to transmit the imaging signal to the signal processing unit 42.

The signal processing unit 42 has a memory that transitorily records the imaging signal received from the communication I/F 41 built therein, and performs processing (image processing, such as demosaicing processing or gamma-correction processing) on the captured image signal that is a set of the imaging signals recorded in the memory to generate captured image data in such a format that recognition processing to be described below can be performed. The captured image data generated by the signal processing unit 42 is recorded on the recording medium 45, such as a hard disk or a flash memory.

The display controller 43 displays a captured image based on the captured image data generated by the signal processing unit 42 on the display device 7. A coordinate of each pixel data constituting the captured image data generated by the signal processing unit 42 is managed in association with a coordinate of any of the display pixels constituting the display surface of the display device 7.

The system control unit 44 controls each unit of the control device 4, and transmits the command to the imaging control unit 27 of the endoscope 1 and the light source processor 51 of the light source device 5, and integrally controls the entire endoscope apparatus 100. For example, the system control unit 44 performs the control of the imaging element 23 via the imaging control unit 27. In addition, the system control unit 44 performs the control of the light source unit 52 via the light source processor 51.

The system control unit 44 includes various processors that execute a program to perform processing, a RAM, and a read only memory (ROM).

Examples of various processors include a central processing unit (CPU), which is a general-purpose processor that executes the program to perform various pieces of processing, a programmable logic device (PLD), which is a processor of which the circuit configuration can be changed after the manufacture, such as a field programmable gate array (FPGA), and a dedicated electric circuit, which is a processor having the circuit configuration specially designed for executing specific processing, such as an application specific integrated circuit (ASIC).

More specifically, the structure of these various processors is an electric circuit in which circuit elements, such as semiconductor elements, are combined.

The system control unit 44 may be composed of one of the various processors, or may be composed of a combination (for example, a combination of a plurality of FPGAs or a combination of a CPU and an FPGA) of two or more processors of the same type or different types.

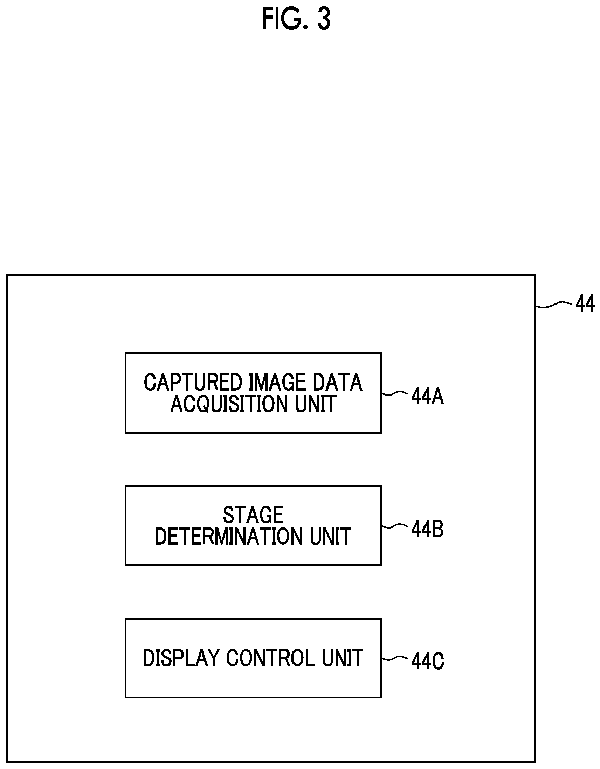

Fig. 3 is a diagram showing an example of functional blocks of the system control unit 44 of the control device 4 shown in Fig. 2.

A processor of the system control unit 44 executes an examination support program stored in the ROM built in the system control unit 44 to function as a display control device comprising a captured image data acquisition unit 44A, a stage determination unit 44B, and a display control unit 44C.

The captured image data acquisition unit 44A sequentially acquires the captured image data obtained by processing the imaging signal, which is obtained by imaging the inside of the subject by the imaging element 23, by the signal processing unit 42.

The stage determination unit 44B determines a stage of the examination using the endoscope 1 based on various pieces of information. The stage determination of the examination based on various pieces of information by the stage determination unit 44B may be the determination by a specific algorithm based on various pieces of information, or may be the determination by a learning model trained through machine learning by a combination of various pieces of information and the information of a search stage.

Examples of the stage of the examination using the endoscope 1 include an insertion stage in which the insertion part 10 of the endoscope 1 is inserted into the subject and a pulling-out stage in which the insertion part 10 of the endoscope 1 is pulled out from the subject. The insertion stage is an example of a first stage according to the embodiment of the present invention. The pulling-out stage is an example of a second stage according to the embodiment of the present invention. In addition, the stage of the examination using the endoscope 1 may include a stage before the insertion part 10 of the endoscope 1 is inserted into the subject or a stage after the insertion part 10 of the endoscope 1 is pulled out from the subject.

For example, the stage determination unit 44B determines the stage of the examination based on the captured image data obtained by the captured image data acquisition unit 44A. Specifically, in general, in the examination of the inside of a large intestine using the endoscope, the insertion part 10 of the endoscope 1 is inserted into a cecum, and then the insertion part 10 of the endoscope 1 is started to be pulled out. Therefore, it can be determined that the stage of the examination is the insertion stage until the cecum is detected based on the captured image data, and the stage of the examination is the pulling-out stage after the cecum is detected based on the captured image data.

The display control unit 44C issues the command to the display controller 43 to control a screen to be displayed on the display device 7. Specifically, the display control unit 44C displays the screen including information in accordance with the determination result of the stage of the examination by the stage determination unit 44B on the display device 7.

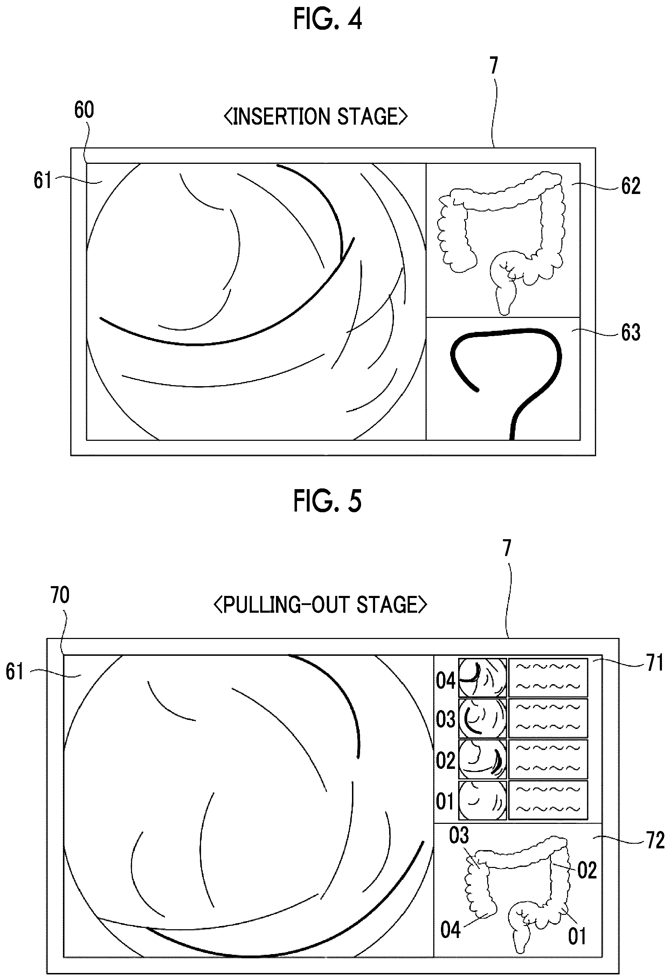

Fig. 4 is a diagram showing an example of the screen displayed on the display device 7 in the insertion stage of the endoscope 1 in the first embodiment.

In a case in which the stage determination unit 44B determines that the stage of the examination is the insertion stage of the endoscope apparatus 100, the display control unit 44C displays, for example, a screen 60 shown in Fig. 4 on the display device 7. The screen 60 includes a live image 61, a large intestine map 62, and an insertion shape image 63.

The left side of the screen 60 is a main region, and the live image 61 is disposed in the main region. The right side of the screen 60 is a sub-region, and the large intestine map 62 and the insertion shape image 63 are disposed in the sub-region.

The live image 61 is a motion picture showing an image obtained by the imaging signal continuously output from the imaging element 23 in real time.

The large intestine map 62 and the insertion shape image 63 constitute insertion support information for supporting the insertion of the insertion part 10 of the endoscope 1 into the subject. The large intestine map 62 is an example of a map of the inside of the subject which is an insertion target of the endoscope 1, and is an image showing the large intestine of the subject which is the insertion target of the endoscope 1. The large intestine map 62 may be, for example, an image obtained by imaging the inside of the subject, or may be a general image of the large intestine prepared in advance.

The insertion shape image 63 is an image showing the insertion shape of the insertion part 10 of the endoscope 1 inserted into the subject. For example, the insertion shape image 63 is an image generated based on a detection result of a magnetic field generated from a magnetic field generation element provided in the distal end part 10C of the endoscope 1. In this case, for example, the endoscope apparatus 100 further comprises a magnetic field detection device that detects the magnetic field from the magnetic field generation element of the endoscope 1 inserted into the subject from outside the subject, and the system control unit 44 generates the insertion shape image 63 based on the detection result by the magnetic field detection device.

In addition, the insertion shape image 63 may be an image generated based on the detection result of the magnetic field by the magnetic field detection element provided in the distal end part 10C of the endoscope 1. In this case, for example, the endoscope apparatus 100 further comprises a magnetic field generation element that generates the magnetic field from outside the subject to the inside of the subject, and the system control unit 44 generates the insertion shape image 63 based on the detection result of the magnetic field by the magnetic field detection element provided in the endoscope 1.

An operator of the endoscope 1 can easily insert the insertion part 10 of the endoscope 1 into the subject by looking at the large intestine map 62 and the insertion shape image 63.

Fig. 5 is a diagram showing an example of a screen displayed on the display device 7 in the pulling-out stage of the endoscope 1 in the first embodiment.

In a case in which the stage determination unit 44B determines that the stage of the examination is the pulling-out stage of the endoscope apparatus 100, the display control unit 44C displays, for example, a screen 70 shown in Fig. 5 on the display device 7. The screen 70 includes the live image 61, thumbnail information 71, and a large intestine imaging map 72.

The live image 61 is disposed in the main region as in the example of Fig. 4. The thumbnail information 71 and the large intestine imaging map 72 are disposed in the sub-region (region on the left side) in place of the large intestine map 62 and the insertion shape image 63 shown in Fig. 4.

The thumbnail information 71 and the large intestine imaging map 72 constitute observation support information for supporting the observation of the captured image obtained by the endoscope 1. The observation support information may be, for example, information for supporting a diagnosis based on the captured image obtained by the endoscope 1.

The thumbnail information 71 is information indicating a plurality of still pictures, which are obtained during the insertion of the endoscope 1 into the subject or during pulling-out of the endoscope 1 from the subject, in a reduced manner. In the example shown in Fig. 5, the thumbnail information 71 includes four still pictures, and the consecutive numbers "01", "02", "03", and "04" are attached to the still pictures.

In addition, the thumbnail information 71 may include other text information (portion of "~~~~" in Fig. 5) relating to each still picture, as in the example shown in Fig. 5. Examples of the text information include a position or a time at which the corresponding still picture is obtained, the findings input by the operator in a case in which the corresponding still picture is obtained, and a result of the image analysis in a case in which the corresponding still picture is obtained.

The large intestine imaging map 72 indicates a portion in which the still pictures described above are obtained in the large intestine map 62 shown in Fig. 4 by the consecutive numbers described above.

By looking at the thumbnail information 71 and the large intestine imaging map 72, the operator of the endoscope 1 can easily observe the captured image obtained by the endoscope 1 or make a diagnosis based on the captured image obtained by the endoscope 1.

In addition, as shown in Figs. 4 and 5, the system control unit 44 may display the screen including the live image 61 and the insertion support information on the display device 7 in the insertion stage, and may display the screen including the live image 61 and the observation support information on the display device 7 in the pulling-out stage. As a result, in the examination mainly performed while observing the live image 61, it is possible to display each support information useful in each of the insertion stage and the insertion stage while displaying the live image 61.

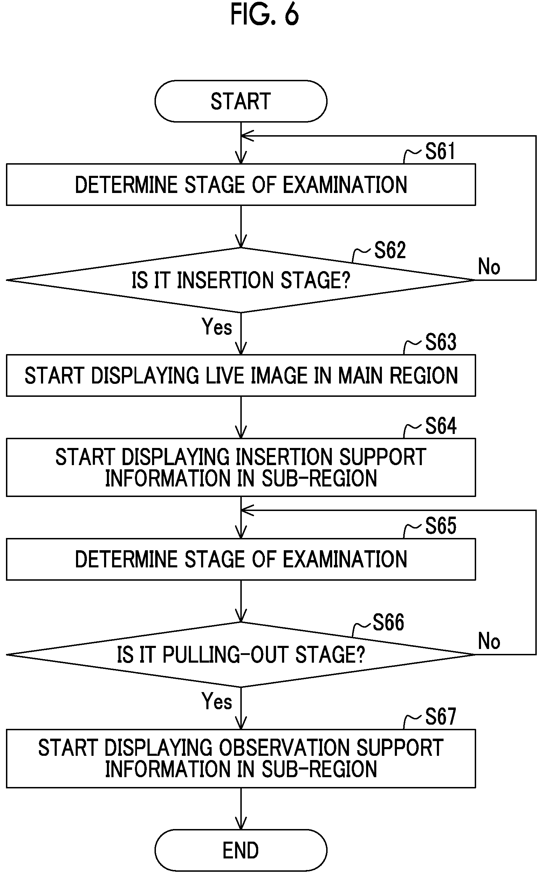

Fig. 6 is a flowchart showing an example of display control processing by the control device 4 in the first embodiment. In a case of the examination using the endoscope 1, the control device 4 executes, for example, the processing shown in Fig. 6.

First, the control device 4 determines the stage of the examination using the endoscope 1 (step S61). Then, the control device 4 determines, based on the result of step S61, whether or not the stage of the examination using the endoscope 1 is the insertion stage in which the endoscope 1 is inserted into the subject (step S62). In a case in which it is not the insertion stage (step S62: No), the control device 4 returns to step S61.

In step S62, in a case in which it is the insertion stage (step S62: Yes), the control device 4 starts displaying the live image 61 in the main region (region on the left side) of the display device 7 (step S63). In addition, the control device 4 starts displaying the insertion support information (large intestine map 62 and insertion shape image 63) in the sub-region (region on the right side) (step S64).

Then, the control device 4 determines the stage of the examination using the endoscope 1 (step S65). Then, the control device 4 determines, based on the result of step S65, whether or not the stage of the examination using the endoscope 1 is the pulling-out stage in which the endoscope 1 is pulled out from the subject (step S66). In a case in which it is not the pulling-out stage (step S66: No), the control device 4 returns to step S65.

In step S66, in a case in which it is the pulling-out stage (step S66: Yes), the control device 4 starts displaying the observation support information (thumbnail information 71 and large intestine imaging map 72) in the sub-region (step S67), and terminates a series of processing.

As described above, the endoscope apparatus 100 according to the first embodiment determines the stage of the examination by the endoscope 1, and then displays the screen including the insertion support information for supporting the insertion of the endoscope 1 into the subject in a case in which it is the insertion stage, and displays the screen including the observation support information for supporting the observation of the image obtained by the endoscope 1 in a case in which it is the pulling-out stage. As a result, it is possible to provide the useful information in accordance with the stage of the examination to the operator of the endoscope.

### <Modification Example of Determination Method of Stage of Examination>

The configuration has been described in which the stage determination unit 44B determines the stage of the examination based on the captured image data, but the present invention is not limited to such a configuration.

For example, the stage determination unit 44B may determine the stage of the examination based on the detection result of the magnetic field generated from the magnetic field generation element provided in the distal end part 10C of the endoscope 1. In this case, for example, the endoscope apparatus 100 further comprises the magnetic field detection device that detects the magnetic field from the magnetic field generation element of the endoscope 1 inserted into the subject from outside the subject, and the stage determination unit 44B determines the stage of the examination based on the detection result by the magnetic field detection device.

In addition, the stage determination unit 44B may determine the stage of the examination based on the detection result of the magnetic field by the magnetic field detection element provided in the distal end part 10C of the endoscope 1. In this case, for example, the endoscope apparatus 100 further comprises the magnetic field generation element that generates the magnetic field from outside the subject to the inside of the subject, and the stage determination unit 44B determines the stage of the examination based on the detection result of the magnetic field by the magnetic field detection element provided in the endoscope 1.

In addition, the stage determination unit 44B may determine the stage of the examination based on acceleration information obtained by an accelerometer provided in the distal end part 10C of the endoscope 1. For example, in the insertion stage, the endoscope 1 moves toward the distal end of the endoscope 1, and in the pulling-out stage, the endoscope 1 moves in a direction opposite to a direction of the distal end of the endoscope 1. Therefore, by specifying a moving direction of the endoscope 1 based on the acceleration information described above, it is possible to determine whether the stage of the examination is the insertion stage or the pulling-out stage.

In addition, in a case in which the endoscope 1 can switch the illumination light having different emission spectra and perform the irradiation, the stage determination unit 44B may determine the stage of the examination based on switching of the illumination light emitted by the endoscope 1. For example, in the light source device 5 shown in Fig. 2, the white light and special light for image enhancement can be switched.

This switching may be performed by a light source switching operation by the operator of the endoscope 1, or may be automatically performed based on the captured image data or the like obtained by the captured image data acquisition unit 44A described above. Generally, the white light is used in the insertion stage and the special light is used in the pulling-out stage, so that it is possible to determine the stage of the examination based on the switching of the illumination light emitted by the endoscope 1.

### <Modification Example of Insertion Support Information>

the configuration has been described in which the system control unit 44 displays the map (large intestine map 62) of the inside of the subject and the image (insertion shape image 63) of the insertion shape of the endoscope 1 as the information for supporting the insertion of the endoscope 1 into the subject, but the present invention is not limited to such a configuration.

For example, the system control unit 44 may display any of the map (large intestine map 62) of the inside of the subject and the image (insertion shape image 63) of the insertion shape of the endoscope 1 as the information for supporting the insertion of the endoscope 1 into the subject, on the display device 7.

Alternatively, the system control unit 44 may display an elapsed time from the start of the insertion of the endoscope 1 into the subject as the information for supporting the insertion of the endoscope 1 into the subject, on the display device 7. As a result, the operator of the endoscope 1 can perform the insertion operation while easily grasping the load on the subject accompanied by the insertion of the endoscope 1.

### <Modification Example of Observation Support Information>

The configuration has been described in which the system control unit 44 displays the still picture (thumbnail information 71) obtained by the imaging element 23 provided in the endoscope 1 and the information (large intestine imaging map 72) indicating the position inside the subject at which the still picture is obtained as the information for supporting the observation, but the present invention is not limited to such a configuration.

For example, a configuration may be adopted in which the system control unit 44 displays only the still picture (thumbnail information 71) obtained by the imaging element 23 provided in the endoscope 1 as the information for supporting the observation on the display device 7. In addition, the thumbnail information 71 does not have to include the text information or the consecutive numbers described above.

In addition, a configuration may be adopted in which the system control unit 44 displays various pieces of information detected based on the image obtained by the imaging element 23 provided in the endoscope 1 on the display device 7 as the information for supporting the observation.

The information detected based on the image obtained by the imaging element 23 is, for example, information on a region-of-interest. The information on the region-of-interest is, for example, an image of a lesion, such as a tumor, an outline of the lesion, or the like. Alternatively, the information on the region-of-interest may be information, such as the number or size of detected lesions.

In addition, the information detected based on the image obtained by the imaging element 23 may be a similar case image selected based on the image obtained by the imaging element 23. For example, the captured images of various past cases are registered in an internal or external database of the endoscope apparatus 100. The system control unit 44 searches the database described above for an image similar to the image obtained by the imaging element 23, and displays the image obtained by the search on the display device 7 as the similar case image.

In addition, a configuration may be adopted in which the system control unit 44 displays information indicating the tumor that is likely to occur in the site inside the subject into which the endoscope 1 is being inserted on the display device 7 as the information for supporting the observation. For example, for each site of the large intestine, the name of the tumor that is likely to occur or the like is registered in the internal or external database of the endoscope apparatus 100. The system control unit 44 specifies the site inside the large intestine into which the endoscope 1 is being inserted, searches the database described above for the name of the tumor that is likely to occur or the like for the specified site, and displays the name of the tumor or the like obtained by the search on the display device 7. The site inside the large intestine into which the endoscope 1 is being inserted can be specified by using, for example, the image obtained by the imaging element 23 or a combination of the magnetic field generation element and the magnetic field detection device described above.

In addition, a configuration may be adopted in which the system control unit 44 displays information indicating a recommended observation method of the site inside the subject into which the endoscope 1 is being inserted on the display device 7 as the information for supporting the observation. For example, for each site of the large intestine, the recommended observation method of the live image is registered in the internal or external database of the endoscope apparatus 100. The system control unit 44 specifies the site inside the large intestine into which the endoscope 1 is being inserted, searches the database described above for the recommended observation method of the specified site, and displays the observation method obtained by the search on the display device 7. The site inside the large intestine into which the endoscope 1 is being inserted can be specified by using, for example, the image obtained by the imaging element 23 or a combination of the magnetic field generation element and the magnetic field detection device described above.

In addition, a configuration may be adopted in which the system control unit 44 displays information indicating a past examination result of the inside of the subject on the display device 7 as the information for supporting the observation. For example, the system control unit 44 acquires the information indicating the result of the examination performed in the past on the subject into which the endoscope 1 is being inserted from the internal or external database of the endoscope apparatus 100, and displays the acquired information indicating the result of the examination on the display device 7. Examples of the information indicating the result of the examination include the image obtained by imaging in the examination, a result of analysis based on the image, or the like.

In addition, the system control unit 44 may display information indicating an elapsed time from the start of pulling-out of the endoscope 1 from the subject on the display device 7 as the information for supporting the observation. As a result, the operator of the endoscope 1 can perform the pulling-out operation while easily grasping the load on the subject accompanied by the pulling-out of the endoscope 1.

### <Modification Example of Insertion Support Information and Observation Support Information>

Alternatively, the system control unit 44 may display a screen including an image obtained by the imaging element 23 in a state in which the inside of the subject is irradiated with the white light on the display device 7 as the information for supporting the insertion in the insertion stage, and may display a screen including an image obtained by the imaging element 23 in a state in which the inside of the subject is irradiated with the special light having a different emission spectrum from the white light on the display device 7 as the information for supporting the observation in the pulling-out stage. The image obtained by the imaging element 23 in a state in which the inside of the subject is irradiated with the special light is an image suitable for the observation or diagnosis described above.

For example, in the insertion stage, the system control unit 44 controls the light source device 5 to emit the white light, and displays the screen including the live image 61 based on the imaging signal obtained by the imaging element 23 on the display device 7. In addition, in the pulling-out stage, the system control unit 44 controls the light source device 5 to emit the special light, and displays the screen including the live image 61 based on the imaging signal obtained by the imaging element 23 on the display device 7. A configuration of the light source device 5 capable of switching the illumination light having different emission spectra and performing the irradiation will be described with reference to Figs. 10 and 11.

### (Second Embodiment)

A different portion of the second embodiment from the first embodiment will be described. In the first embodiment, the configuration has been described in which the insertion stage and the pulling-out stage are determined and the useful information is displayed in each of these stages. In the second embodiment, a configuration will be described in which it is determined whether the stage is the stage in which the insertion part 10 of the endoscope 1 is inserted into any site inside the subject, and the useful information is displayed in each of these stages.

Here, an examination performed by inserting the insertion part 10 of the endoscope 1 into a stomach from a mouth of the subject via an esophagus will be described. In this case, examples of the stage of the examination determined by the stage determination unit 44B include an esophagus insertion stage in which the insertion part 10 of the endoscope 1 is inserted into the esophagus, and a stomach insertion stage in which the insertion part 10 of the endoscope 1 is inserted into the stomach. The esophagus is an example of a first site inside the subject. The stomach is an example of a second site inside the subject.

For example, the stage determination unit 44B determines the esophagus insertion stage and the stomach insertion stage based on the captured image data obtained by the captured image data acquisition unit 44A.

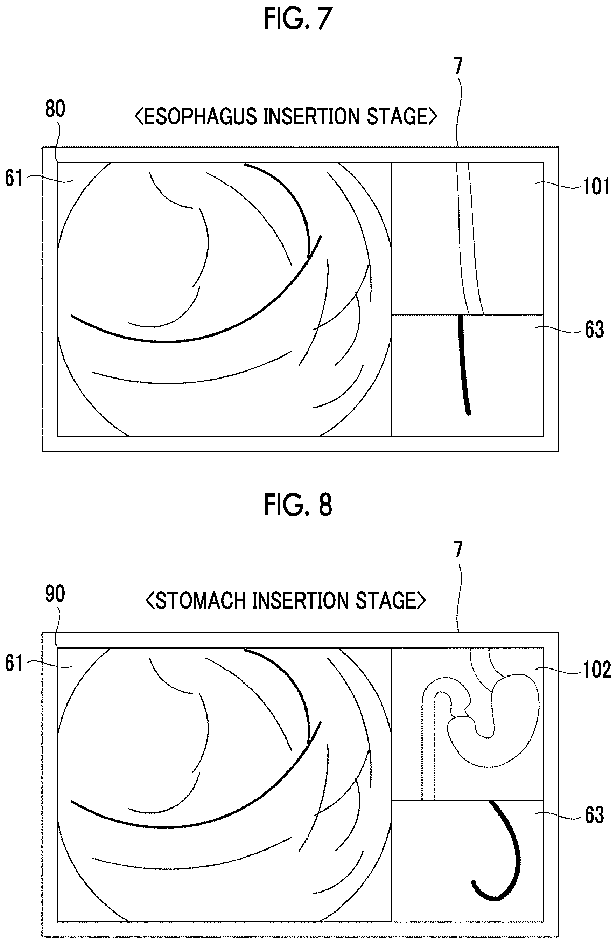

Fig. 7 is a diagram showing an example of a screen displayed on the display device 7 in the esophagus insertion stage of the endoscope 1 in the second embodiment.

In a case in which the stage determination unit 44B determines that the stage of the examination is the esophagus insertion stage of the endoscope apparatus 100, the display control unit 44C displays, for example, a screen 80 shown in Fig. 7 on the display device 7. The screen 80 includes the live image 61, an esophagus map 101, and the insertion shape image 63.

The live image 61 is disposed in the main region (region on the left side). The esophagus map 101 and the insertion shape image 63 are disposed in the sub-region (region on the right side).

The live image 61 is a motion picture showing an image obtained by the imaging signal continuously output from the imaging element 23 in real time.

The esophagus map 101 and the insertion shape image 63 constitute esophagus insertion support information for supporting the insertion of the insertion part 10 of the endoscope 1 into the esophagus.

The esophagus map 101 is an image indicating the esophagus of the subject which is the insertion target of the endoscope 1. The esophagus map 101 may be, for example, an image obtained by imaging the inside of the subject, or may be a general image of the large intestine prepared in advance. In the example shown in Fig. 7, the insertion shape image 63 indicates the shape of the insertion part 10 of the endoscope 1 inserted into the esophagus inside the subject.

By looking at the esophagus map 101 and the insertion shape image 63, the operator of the endoscope 1 can easily insert the insertion part 10 of the endoscope 1 into the esophagus.

Fig. 8 is a diagram showing an example of a screen displayed on the display device 7 in the stomach insertion stage of the endoscope 1 in the second embodiment.

In a case in which the stage determination unit 44B determines that the stage of the examination is the stomach insertion stage of the endoscope apparatus 100, the display control unit 44C displays, for example, a screen 90 shown in Fig. 8 on the display device 7. The screen 90 includes the live image 61, a stomach map 102, and the insertion shape image 63.

The live image 61 is disposed in the main region as in the example of Fig. 7. The stomach map 102 and the insertion shape image 63 are disposed in the sub-region in place of the esophagus map 101 and the insertion shape image 63 shown in Fig. 7.

The stomach map 102 and the insertion shape image 63 constitute stomach insertion support information for supporting the insertion of the insertion part 10 of the endoscope 1 into the stomach inside the subject.

The stomach map 102 is an image indicating the stomach of the subject which is the insertion target of the endoscope 1. The stomach map 102 may be, for example, an image obtained by imaging the inside of the subject, or may be a general image of the large intestine prepared in advance. In the example shown in Fig. 8, the insertion shape image 63 indicates the shape of the insertion part 10 of the endoscope 1 inserted into the stomach inside the subject.

By looking at the stomach map 102 and the insertion shape image 63, the operator of the endoscope 1 can easily insert the insertion part 10 of the endoscope 1 into the stomach.

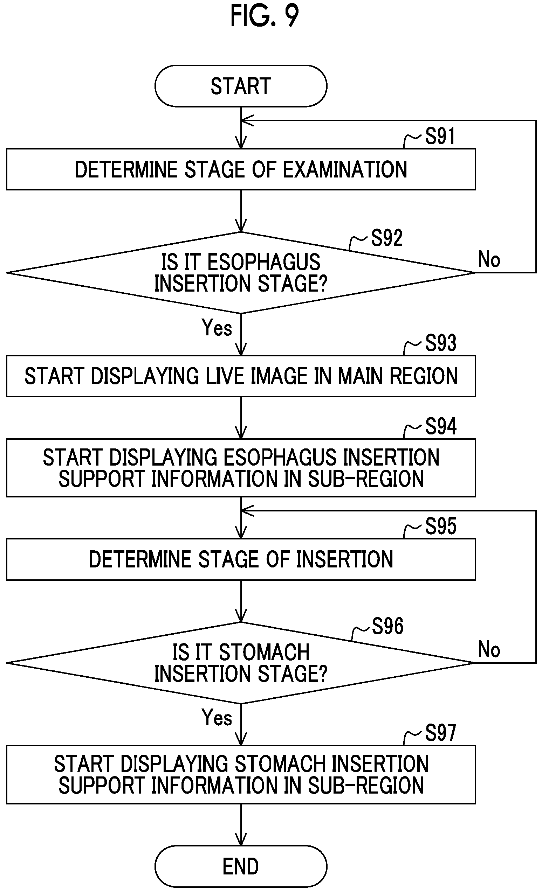

Fig. 9 is a flowchart showing an example of the display control processing of the control device 4 in the second embodiment. In a case of the examination using the endoscope 1, the control device 4 executes, for example, the processing shown in Fig. 9.

First, the control device 4 determines the stage of the examination using the endoscope 1 (step S91). Then, the control device 4 determines, based on the result of step S91, whether or not the stage of the examination using the endoscope 1 is the esophagus insertion stage in which the endoscope 1 is inserted into the esophagus of the subject (step S92). In a case in which it is not the esophagus insertion stage (step S92: No), the control device 4 returns to step S91.

In step S92, in a case in which it is the esophagus insertion stage (step S92: Yes), the control device 4 starts displaying the live image 61 in the main region (region on the left side) of the display device 7 (step S93). In addition, the control device 4 starts displaying the esophagus insertion support information (esophagus map 101 and insertion shape image 63) in the sub-region (region on the right side) (step S94).

Then, the control device 4 determines the stage of the examination using the endoscope 1 (step S95). Then, the control device 4 determines, based on the result of step S95, whether or not the stage of the examination using the endoscope 1 is the stomach insertion stage in which the endoscope 1 is inserted into the stomach of the subject (step S96). In a case in which it is not the stomach insertion stage (step S96: No), the control device 4 returns to step S95.

In step S96, in a case in which it is the stomach insertion stage (step S96: Yes), the control device 4 starts displaying the stomach insertion support information (stomach map 102 and insertion shape image 63) in the sub-region (step S97), and terminates a series of processing.

As described above, the endoscope apparatus 100 of the second embodiment determines the stage of the examination by the endoscope 1, and then displays the screen including the esophagus insertion support information for supporting the insertion of the endoscope 1 into the esophagus in a case in which it is the esophagus insertion stage, and displays the screen including the stomach insertion support information for supporting the insertion of the endoscope 1 into the stomach in a case in which it is the stomach insertion stage. As a result, it is possible to provide the useful information in accordance with the stage of the examination to the operator of the endoscope.

### <Modification Example of First Site and Second Site>

Although the esophagus and the stomach have been described as examples of the first site and the second site, the first site and the second site are not limited to these. For example, in a case in which the large intestine is examined using the endoscope 1, the first site and the second site may be two sites among a sigmoid colon, a descending colon, a transverse colon, and an ascending colon. In addition, the number of sites in which the insertion is determined may be three or more, and the system control unit 44 may display information for supporting the insertion of the insertion part 10 into the target site for each site in which the insertion is determined on the display device 7.

### <Modification Example of Determination Method of Stage of Examination>

The configuration has been described in which the stage determination unit 44B determines the stage of the examination (insertion site) based on the captured image data, but the present invention is not limited to such a configuration.

For example, the stage determination unit 44B may determine the stage of the examination based on the detection result of the magnetic field generated from the magnetic field generation element provided in the distal end part 10C of the endoscope 1. In this case, for example, the endoscope apparatus 100 further comprises the magnetic field detection device that detects the magnetic field from the magnetic field generation element of the endoscope 1 inserted into the subject from outside the subject, and the stage determination unit 44B determines the stage of the examination based on the detection result by the magnetic field detection device.

In addition, the stage determination unit 44B may determine the stage of the examination based on the detection result of the magnetic field by the magnetic field detection element provided in the distal end part 10C of the endoscope 1. In this case, for example, the endoscope apparatus 100 further comprises the magnetic field generation element that generates the magnetic field from outside the subject to the inside of the subject, and the stage determination unit 44B determines the stage of the examination based on the detection result of the magnetic field by the magnetic field detection element provided in the endoscope 1.

### (Combination of First and Second Embodiments)

The first and second embodiments may be combined. That is, the system control unit 44 may determine, for example, the insertion stage and the pulling-out stage for each of a plurality of sites inside the subject, and may display the insertion support information or the observation support information for each site during the insertion on the display device 7 in accordance with the determined stage.

### (About Light Source Device 5)

Fig. 10 is a diagram showing an example of the light source device 5 capable of switching the illumination light having different emission spectra and performing the irradiation. Fig. 11 is a diagram showing an example of a spectrum of the light generated by the light source device 5 shown in Fig. 10.

As shown in Fig. 10, the light source device 5 shown in Fig. 2 comprises the light source processor 51, the light source unit 52, and an optical path coupling unit 54.

The light source unit 52 has, for example, a plurality of semiconductor light sources, each of which is turned on or off, and in a case in which the light source unit 52 is turned on, the emission amount of each semiconductor light source is controlled to emit the illumination light for illuminating an observation target. In the present embodiment, the light source unit 52 has LEDs of four colors, a violet light emitting diode (V-LED) 52a, a blue light emitting diode (B-LED) 52b, a green light emitting diode (G-LED) 52c, and a red light emitting diode (R-LED) 52d.

As shown in Fig. 11, the V-LED 52a generates violet light V of which a central wavelength is in a range of 405±10 nm and a wavelength range is in a range of 380 to 420 nm. The B-LED 52b generates blue light B of which a central wavelength is in a range of 450±10 nm and a wavelength range is in a range of 420 to 500 nm. The G-LED 52c generates green light G of which a wavelength range is in a range of 480 to 600 nm. The R-LED 52d generates red light R of which a central wavelength is in a range of 620 to 630 nm and a wavelength range is in a range of 600 to 650 nm.

The light source processor 51 controls the V-LED 52a, the B-LED 52b, the G-LED 52c, and the R-LED 52d. By independently controlling each of the LEDs 52a to 52d, the light source processor 51 can emit the violet light V, the blue light B, the green light G, or the red light R by independently changing a light amount. In addition, in a normal observation mode, the light source processor 51 controls each of the LEDs 52a to 52d to emit the white light in which a light amount ratio of the violet light V, the blue light B, the green light G, and the red light R is Vc:Bc:Gc:Rc. It should be noted that Vc, Bc, Gc, Rc > 0.

In addition, in a special observation mode, the light source processor 51 controls each of the LEDs 52a to 52d to emit the special light in which the light amount ratio of the violet light V, the blue light B, the green light G, and the red light R as short-wavelength narrow band light is Vs:Bs:Gs:Rs. The light amount ratio Vs:Bs:Gs:Rs is different from the light amount ratio Vc:Bc:Gc:Rc used in the normal observation mode, and is appropriately determined in accordance with the observation purpose. For example, in a case in which superficial blood vessels are enhanced, it is preferable to make Vs larger than Bs, Gs, and Rs, and in a case in which mesopelagic blood vessels are enhanced, it is preferable to make Gs larger than Vs, Gs, and Rs.

With the configuration of the light source device 5 shown in Figs. 10 and 11, it is possible to switch the illumination light having different emission spectra and perform the irradiation in accordance with the command from the control device 4.

### (Control Program)

A control program, which is stored in the ROM of the control device 4, is stored in a program computer-readable non-transitory storage medium. Examples of such a "computer-readable storage medium" include an optical medium, such as a compact disc-ROM (CD-ROM), and a magnetic storage medium, such as a universal serial bus (USB) memory, or a memory card. In addition, such a program can also be provided by being downloaded via a network.

### (Another Embodiment of Endoscope System)

The endoscope apparatus 100 has been described as an example of the endoscope system according to the embodiment of the present invention, the endoscope system according to the embodiment of the present invention may be realized by a plurality of devices connected to each other via the network. For example, a configuration may be adopted in which at least a part of the processing by the control device 4 described above is executed by another device connected to the endoscope apparatus 100 via the network.

### (Another Embodiment of Display Unit)

Although the display device 7 has been described as an example of the display unit according to the embodiment of the present invention, the display unit according to the embodiment of the present invention may be realized by a plurality of display devices. In this case, the main region described above may be composed of one display device of the plurality of display devices, and the sub-region described above may be composed of the remaining display devices of the plurality of display devices.

According to the present invention, it is possible to provide the endoscope system and the control program that can provide useful information in accordance with the stage of the examination to the operator of the endoscope.

### Explanation of References

1: endoscope
4: control device
5: light source device
6: input unit
7: display device
10: insertion part
10A: flexible part
10B: bendable part
10C: distal end part
11: operating part
12: angle knob
13: universal cord
13A, 13B: connector portion
21: objective lens
22: lens group
23: imaging element
24: ADC
25: memory
26, 41: communication I/F
27: imaging control unit
42: signal processing unit
43: display controller
44: system control unit
44A: captured image data acquisition unit
44B: stage determination unit
44C: display control unit
45: recording medium
50: illumination lens
51: light source processor
52: light source unit
52a: V-LED
52b: B-LED
52c: G-LED
52d: R-LED
53: light guide
54: optical path coupling unit
60, 70, 80, 90: screen
61: live image
62: large intestine map
63: insertion shape image
71: thumbnail information
72: large intestine imaging map
100: endoscope apparatus
101: esophagus map
102: stomach map

## Claims

1. An endoscope system comprising:
a determination unit (44B) that determines a stage of an examination by an endoscope; and
a display unit (44C) that switches a display image in accordance with a determination result by the determination unit,
wherein the stage of the examination includes a first stage in which the endoscope is being inserted into a subject, and a second stage in which the endoscope is being pulled out from the subject, and
the display unit displays a screen (60, 70, 80, 90) including information for supporting insertion of the endoscope into the subject in a case in which it is determined that the stage of the examination is the first stage, and displays a screen including information for supporting observation of an image obtained by the endoscope in a case in which it is determined that the stage of the examination is the second stage,
wherein the information for supporting the insertion includes an image of an insertion shape of the endoscope and a body part map (62) of an inside of a body part of the subject which is the insertion target of the endoscope, the body part map (62) being an image of the inside of the body part, and the information for supporting the observation includes thumbnail information (71) comprising one or more still images obtained by the imaging element provided in the endoscope and a body part imaging map (72) indicating a portion in which the thumbnail information still images are obtained in the body part map (62) of the inside of the subject.

2. The endoscope system according to claim 1,
wherein the determination unit determines the stage of the examination based on an image obtained by an imaging element provided in the endoscope; and/or
wherein the determination unit determines the stage of the examination based on a detection result of a magnetic field generated from a magnetic field generation element provided in the endoscope; and/or
wherein the determination unit determines the stage of the examination based on a detection result of a magnetic field by a magnetic field detection element provided in the endoscope; and/or
wherein the determination unit determines the stage of the examination based on acceleration information obtained by an accelerometer provided in the endoscope.

3. The endoscope system according to claim 1 or 2,
wherein the endoscope is able to switch illumination light having different emission spectra to perform irradiation, and
the determination unit determines the stage of the examination based on switching of the illumination light emitted by the endoscope.

4. The endoscope system according to any one of claims 1 to 3,
wherein the display unit displays a screen including a live image (61) obtained by an imaging element provided in the endoscope and the information for supporting the insertion in a case in which it is determined that the stage of the examination is the first stage, and displays a screen including the live image and the information for supporting the observation in a case in which it is determined that the stage of the examination is the second stage.

5. The endoscope system according to any one of claims 1 to 4,
wherein the information for supporting the insertion includes information indicating an elapsed time from start of the insertion of the endoscope into the subject; and/or
wherein the information for supporting the observation includes a still picture obtained by an imaging element provided in the endoscope; and/or
wherein the information for supporting the observation includes information on a region-of-interest detected based on an image obtained by an imaging element provided in the endoscope; and/or
wherein the information for supporting the observation includes a similar case image selected based on an image obtained by an imaging element provided in the endoscope; and/or
wherein the information for supporting the observation includes information indicating a tumor that is likely to occur at a site inside the subject into which the endoscope is being inserted; and/or
wherein the information for supporting the observation includes information indicating a recommended observation method of a site inside the subject into which the endoscope is being inserted; and/or
wherein the information for supporting the observation includes information indicating a past examination result of an inside of the subject; and/or
wherein the information for supporting the insertion includes an image obtained by an imaging element provided in the endoscope in a state in which an inside of the subject is irradiated with white light, and
the information for supporting the observation includes an image obtained by the imaging element in a state in which the inside of the subject is irradiated with light having a different emission spectrum from the white light; and/or
wherein the information for supporting the observation includes information indicating an elapsed time from start of pulling-out of the endoscope from an inside of the subject.

6. A control program causing a computer to function as the determination unit and the display unit of the endoscope system according to any one of claims 1 to 5.

## Patentansprüche

1. Endoskopsystem, umfassend:
eine Bestimmungseinheit (44B), die ein Stadium einer Untersuchung durch ein Endoskop bestimmt; und
eine Anzeigeeinheit (44C), die ein Anzeigebild in Übereinstimmung mit einem Bestimmungsergebnis durch die Bestimmungseinheit umschaltet,
wobei das Stadium der Untersuchung ein erstes Stadium, bei dem das Endoskop in eine Untersuchungsperson eingeführt wird, und ein zweites Stadium, bei dem das Endoskop aus der Untersuchungsperson herausgezogen wird, enthält, und
die Anzeigeeinheit einen Bildschirm (60, 70, 80, 90), der Informationen zum Unterstützen von Einführen des Endoskops in die Untersuchungsperson enthält, in einem Fall anzeigt, in dem bestimmt wird, dass das Stadium der Untersuchung das erste Stadium ist, und einen Bildschirm, der Informationen zum Unterstützen von Beobachtung eines Bildes, das durch das Endoskop erhalten wird, enthält, in einem Fall anzeigt, in dem bestimmt wird, dass das Stadium der Untersuchung das zweite Stadium ist,
wobei die Informationen zum Unterstützen des Einführens ein Bild einer Einführungsform des Endoskops und eine Körperteilkarte (62) eines Inneren eines Körperteils der Untersuchungsperson, die das Einführungsziel des Endoskops ist, enthält, wobei die Körperteilkarte (62) ein Bild des Inneren des Körperteils ist, und die Informationen zum Unterstützen der Beobachtung Vorschaubildinformationen (71), die ein oder mehrere Standbilder, die von dem Bildgebungselement, das bei dem Endoskop vorgesehen ist, erhalten werden, umfassen, und eine Körperteil-Bildgebungskarte (72), die einen Abschnitt, in dem die Standbilder von Vorschaubildinformationen erhalten werden, in der Körperteilkarte (62) des Inneren der Untersuchungsperson angibt, enthalten.

2. Endoskopsystem nach Anspruch 1,
wobei die Bestimmungseinheit das Stadium der Untersuchung auf der Grundlage eines Bildes, das von einem Bildgebungselement, das bei dem Endoskop vorgesehen ist, erhalten wird, bestimmt; und/oder
wobei die Bestimmungseinheit das Stadium der Untersuchung auf der Grundlage eines Detektionsergebnisses eines Magnetfelds, das von einem Magnetfeld-Erzeugungselement, das bei dem Endoskop vorgesehen ist, erzeugt wird, bestimmt; und/oder
wobei die Bestimmungseinheit das Stadium der Untersuchung auf der Grundlage eines Detektionsergebnisses eines Magnetfelds durch ein Magnetfeld-Detektionselement, das bei dem Endoskop vorgesehen ist, bestimmt; und/oder
wobei die Bestimmungseinheit das Stadium der Untersuchung auf der Grundlage von Beschleunigungsinformationen, die von einem Beschleunigungsmesser, der bei dem Endoskop vorgesehen ist, erhalten werden, bestimmt.

3. Endoskopsystem nach Anspruch 1 oder 2,
wobei das Endoskop in der Lage ist, Beleuchtungslicht mit unterschiedlichen Emissionsspektren umzuschalten, um Bestrahlung durchzuführen, und
die Bestimmungseinheit das Stadium der Untersuchung auf der Grundlage von Umschalten des von dem Endoskop emittierten Beleuchtungslichts bestimmt.

4. Endoskopsystem nach einem der Ansprüche 1 bis 3,
wobei die Anzeigeeinheit einen Bildschirm, der ein Livebild (61), das von einem Bildgebungselement, das bei dem Endoskop vorgesehen ist, erhalten wird, und die Informationen zum Unterstützen des Einführens enthält, in einem Fall anzeigt, in dem bestimmt wird, dass das Stadium der Untersuchung das erste Stadium ist, und einen Bildschirm, der das Livebild und die Informationen zum Unterstützen der Beobachtung enthält, in einem Fall anzeigt, in dem bestimmt wird, dass das Stadium der Untersuchung das zweite Stadium ist.

5. Endoskopsystem nach einem der Ansprüche 1 bis 4,
wobei die Informationen zum Unterstützen des Einführens Informationen, die eine verstrichene Zeit seit Beginn des Einführens des Endoskops in die Untersuchungsperson angeben, enthalten; und/oder
wobei die Informationen zum Unterstützen der Beobachtung ein Standbild, das von einem Bildgebungselement, das bei dem Endoskop vorgesehen ist, erhalten wird, enthalten; und/oder
wobei die Informationen zum Unterstützen der Beobachtung Informationen über einen Bereich von Interesse, der auf der Grundlage eines Bildes, das von einem Bildgebungselement, das bei dem Endoskop vorgesehen ist, erhalten wird, detektiert wird, enthalten; und/oder
wobei die Informationen zum Unterstützen der Beobachtung ein ähnliches Fallbild, das auf der Grundlage eines Bildes, das von einem Bildgebungselement, das bei dem Endoskop vorgesehen ist, erhalten wird, ausgewählt wird, enthalten; und/oder
wobei die Informationen zum Unterstützen der Beobachtung Informationen, die einen Tumor angeben, der wahrscheinlich an einer Stelle innerhalb der Untersuchungsperson, in die das Endoskop eingeführt wird, auftritt, enthalten;
und/oder
wobei die Informationen zum Unterstützen der Beobachtung Informationen, die ein empfohlenes Beobachtungsverfahren einer Stelle innerhalb der Untersuchungsperson, in die das Endoskop eingeführt wird, angeben, enthalten; und/oder
wobei die Informationen zum Unterstützen der Beobachtung Informationen, die ein vergangenes Untersuchungsergebnis eines Inneren der Untersuchungsperson angeben, enthalten; und/oder
wobei die Informationen zum Unterstützen des Einführens ein Bild, das von einem Bildgebungselement, das bei dem Endoskop vorgesehen ist, in einem Zustand erhalten wird, in dem ein Inneres der Untersuchungsperson mit Weißlicht bestrahlt wird, enthalten, und
die Informationen zum Unterstützen der Beobachtung ein Bild, das von dem Bildgebungselement in einem Zustand erhalten wird, in dem das Innere der Untersuchungsperson mit Licht, das ein anderes Emissionsspektrum als das Weißlicht aufweist, bestrahlt wird, enthalten; und/oder
wobei die Informationen zum Unterstützen der Beobachtung Informationen, die eine verstrichene Zeit seit Beginn von Herausziehen des Endoskops aus einem Inneren der Untersuchungsperson angeben, enthalten.

6. Steuerprogramm, das einen Computer veranlasst, als die Bestimmungseinheit und die Anzeigeeinheit des Endoskopsystems nach einem der Ansprüche 1 bis 5 zu fungieren.

## Revendications

1. Système d'endoscope comprenant :
une unité de détermination (44B) qui détermine une étape d'un examen par un endoscope ; et
une unité d'affichage (44C) qui commute une image d'affichage en fonction d'un résultat de détermination par l'unité de détermination,
dans lequel l'étape de l'examen inclut une première étape dans laquelle l'endoscope est inséré dans un sujet, et une deuxième étape dans laquelle l'endoscope est retiré du sujet, et
l'unité d'affichage affiche un écran (60, 70, 80, 90) incluant des informations pour faciliter l'insertion de l'endoscope dans le sujet dans un cas où il est déterminé que l'étape de l'examen est la première étape, et affiche un écran incluant des informations pour faciliter l'observation d'une image obtenue par l'endoscope dans un cas où il est déterminé que l'étape de l'examen est la deuxième étape,
dans lequel les informations pour faciliter l'insertion incluent une image d'une forme d'insertion de l'endoscope et une carte de partie de corps (62) d'un intérieur d'une partie de corps du sujet qui est la cible d'insertion de l'endoscope, la carte de partie de corps (62) étant une image de l'intérieur de la partie de corps, et les informations pour faciliter l'observation incluent des informations de vignette (71) comprenant une ou plusieurs images fixes obtenues par l'élément d'imagerie prévu dans l'endoscope et une carte d'imagerie de partie de corps (72) indiquant une partie dans laquelle les images fixes des informations de vignette sont obtenues dans la carte de partie de corps (62) de l'intérieur du sujet.

2. Système d'endoscope selon la revendication 1,
dans lequel l'unité de détermination détermine l'étape de l'examen sur la base d'une image obtenue par un élément d'imagerie prévu dans l'endoscope ; et/ou
dans lequel l'unité de détermination détermine l'étape de l'examen sur la base d'un résultat de détection d'un champ magnétique généré par un élément de génération de champ magnétique prévu dans l'endoscope ; et/ou
dans lequel l'unité de détermination détermine l'étape de l'examen sur la base d'un résultat de détection d'un champ magnétique par un élément de détection de champ magnétique prévu dans l'endoscope ; et/ou
dans lequel l'unité de détermination détermine l'étape de l'examen sur la base d'informations d'accélération obtenues par un accéléromètre prévu dans l'endoscope.

3. Système d'endoscope selon la revendication 1 ou la revendication 2,
dans lequel l'endoscope est capable de commuter une lumière d'éclairage ayant différents spectres d'émission pour effectuer une irradiation, et
l'unité de détermination détermine l'étape de l'examen sur la base de la commutation de la lumière d'éclairage émise par l'endoscope.

4. Système d'endoscope selon l'une quelconque des revendications 1 à 3,
dans lequel l'unité d'affichage affiche un écran incluant une image en direct (61) obtenue par un élément d'imagerie prévu dans l'endoscope et les informations pour faciliter l'insertion dans un cas où il est déterminé que l'étape de l'examen est la première étape, et affiche un écran incluant l'image en direct et les informations pour faciliter l'observation dans un cas où il est déterminé que l'étape de l'examen est la deuxième étape.

5. Système d'endoscope selon l'une quelconque des revendications 1 à 4,
dans lequel les informations pour faciliter l'insertion incluent des informations indiquant un temps écoulé depuis le début de l'insertion de l'endoscope dans le sujet ; et/ou
dans lequel les informations pour faciliter l'observation incluent une image fixe obtenue par un élément d'imagerie prévu dans l'endoscope ; et/ou
dans lequel les informations pour faciliter l'observation incluent des informations sur une région d'intérêt détectée sur la base d'une image obtenue par un élément d'imagerie prévu dans l'endoscope ; et/ou
dans lequel les informations pour faciliter l'observation incluent une image de cas similaire sélectionnée sur la base d'une image obtenue par un élément d'imagerie prévu dans l'endoscope ; et/ou
dans lequel les informations pour faciliter l'observation incluent des informations indiquant une tumeur qui est susceptible de se développer à un site à l'intérieur du sujet dans lequel l'endoscope est inséré ; et/ou
dans lequel les informations pour faciliter l'observation incluent des informations indiquant un mode d'observation recommandé d'un site à l'intérieur du sujet dans lequel l'endoscope est inséré ; et/ou
dans lequel les informations pour faciliter l'observation incluent des informations indiquant un résultat d'examen antérieur d'un intérieur du sujet ; et/ou
dans lequel les informations pour faciliter l'insertion incluent une image obtenue par un élément d'imagerie prévu dans l'endoscope dans un état dans lequel un intérieur du sujet est irradié par une lumière blanche, et
dans lequel les informations pour faciliter l'observation incluent une image obtenue par l'élément d'imagerie dans un état dans lequel l'intérieur du sujet est irradié par une lumière ayant un spectre d'émission différent de celui de la lumière blanche ; et/ou
dans lequel les informations pour faciliter l'observation incluent des informations indiquant un temps écoulé depuis le début du retrait de l'endoscope d'un intérieur du sujet.

6. Programme de contrôle amenant un ordinateur à fonctionner en tant qu'unité de détermination et unité d'affichage du système d'endoscope selon l'une quelconque des revendications 1 à 5.
